# EUROPÄISCHE PATENTSCHRIFT

(11) **EP 0 598 247 B1**
(45) Veröffentlichungstag und Bekanntmachung des Hinweises auf die Patenterteilung: **22.09.1999**
(21) Anmeldenummer: 93117198.7
(22) Anmeldetag: 22.10.1993
(51) Int. Cl.: A61C 19/00, A61L 2/02

(54) **Vorrichtung zum Reinigen und Pflegen von ärztlichen oder zahnärtzlichen Instrumenten**
Device for cleaning and care of medical or dental instruments
Dispositif pour le nettoyage et le traitement d'instruments médicaux ou dentaires

(30) Priorität: 22.10.1992 DE 4235699
(43) Veröffentlichungstag der Anmeldung: 25.05.1994
(60) Teilanmeldung: 99102463.9
(73) Patentinhaber: KALTENBACH & VOIGT GMBH & CO., 88396 Biberach (DE)
(72) Erfinder: Steinhauser, Pius, D-88299 Leutkirch (DE); Bodenmiller, Anton, D-88299 Leutkirch (DE); Lott, Herbert, D-88410 Bad Wurzach (DE)
(74) Vertreter: Schmidt-Evers, Jürgen, Dipl.-Ing.

(56) Entgegenhaltungen:
- EP-A- 0 369 043
- EP-A- 0 403 442
- EP-A- 0 484 628
- EP-A- 0 494 031
- EP-A- 0 499 785
- WO-A-88/02621
- DE-A- 4 024 171
- DE-A- 4 125 223
- FR-A- 2 601 518
- US-A- 4 544 355
- US-A- 4 607 410

## Beschreibung

Die Erfindung bezieht sich auf eine Vorrichtung nach dem Oberbegriff des Anspruchs 1.

Bei vorliegenden Instrumenten oder Handstücken handelt es sich um die rohrförmigen Teile von Bearbeitungsinstrumenten, die der Arzt während der Behandlung als Griffhülse ergreift. Ein vorliegendes Handstück trägt an seinem vorderen Ende ein Behandlungswerkzeug, z.B. einen Bohrer, und ist mit seinem hinteren Ende mittels einer Steck/Dreh-Kupplung mit einem sogenannten Versorgungsteil kuppelbar, dem durch ein Versorgungskabel Energie zum Antrieb des Behandlungswerkzeugs und auch Behandlungsmedien in zugehörigen Fluidleitungen zuführbar sind. Sowohl die Antriebsenergieleitung als auch die Fluidleitungen durchsetzen die Steck/Dreh-Kupplung und setzen sich im Handstück fort. Bei den Fluidleitungen handelt es sich in der Regel um Luft-, Wasser- oder Sprayleitungen, die sich als Kanäle oder Schlauchleitungen bis zum vorderen Ende des Handstücks erstrecken und dort austreten, um auf die Behandlungsstelle einwirken zu können und/oder bis zu einer im Handstück angeordneten Luftturbine. Bei einer Energieleitung kann es sich um eine elektrische Leitung, um eine Fluidleitung, insbesondere Luftleitung, oder auch um eine mechanische Verbindung z.B. in Form einer ein Drehmoment übertragenden Spindel, handeln. Dabei gibt es Behandlungsinstrumente, bei denen der Antriebsmotor im Versorgungsteil oder im Handstück angeordnet ist. Aus baulichen Gründen haben sich in der Praxis solche Konstruktionen durchgesetzt, bei denen ein Elektromotor im Versorgungsteil und ein Luftmotor (bei einer sog. Turbine) im Handstück angeordnet ist. Die Getriebeverbindung zwischen dem Antriebsmotor und dem Behandlungswerkzeug erfolgt durch mechanische Kupplungen, Zahnradverbindungen und Zahnradgetriebe. Bei einem vorliegenden Handstück kann es sich sowohl um ein gerade erstreckendes hülsenförmiges Bauteil als auch um ein sog. Winkelstück handeln. Derzeit übliche Steck/Dreh-Kupplungen zwischen dem Handstück und dem Versorgungsteil werden in den meisten Fällen durch einen am Versorgungsteil angeordneten und von diesem vorspringenden runden Steckzapfen und ein am hinteren Ende des Handstücks angeordnetes, den Steckzapfen aufnehmendes Steckloch gebildet. Ein Handstück der vorliegenden Art umfaßt somit mechanische, pneumatische und/oder hydraulische Funktionselemente.

Bei der Benutzung eines Handstücks zur Behandlung des menschlichen oder tierischen Körpers oder Bearbeitung von prothetischen Teilen, die von Patienten getragen werden, erfolgt zwangsläufig eine Verunreinigung und Kontaminierung durch Krankheitserreger. Es ist deshalb erforderlich, ein Handstück nach der Benutzung zu reinigen und zu desinfizieren. Im Hinblick auf die mechanischen Funktionsteile bedarf es dabei auch einer Pflege der Mechanik, z.B. mit einem besonderen Pflegeöl, um einen ungestörten Lauf und eine lange Lebensdauer der mechanischen Antriebsteile zu gewährleisten.

Eine Vorrichtung, die dies ermöglicht, ist in der DE-40 24 171 A 1 (die alle Merkmale des Oberbegriffs des Anspruchs 1 offenbart) beschrieben. Bei dieser bekannten Ausgestaltung erfolgt die Reinigung, Desinfektion und Pflege wenigstens eines, vorzugsweise mehrerer Handstücke gleichzeitig, in einem Wasserbad in einem Behälter, wobei jedem Handstück eine Halterung im Behälter zugeordnet ist, mit der das zugehörige Handstück in aufrechter Anordnung in Halteverbindung bringbar ist. Jeder Halterung ist eine Druckluftleitung zugeordnet, die in das Innere des zugehörigen Handstücks mündet. Die durch die Druckluftleitung zuführbare Druckluft ist wahlweise beheizbar, so daß heiße Druckluft durch das Handstück geblasen werden kann. Außerdem ist ein Ölvorrat mit einem Öldosierer durch einen Leitungsabschnitt mit der Druckluftleitung verbunden, um wahlweise Öl in den Hohlraum des Handstücks einzuleiten, der die mechanischen Antriebsteile aufnimmt. Bei dieser bekannten Ausgestaltung erfolgt die Reinigung und Desinfektion des Handstücks außen und innen durch Kochen im Wasserbad, wobei auch Reinigungs- und Tensidzusätze in das Wasserbad einführbar sind. Die Innenreinigung und -pflege erfolgt zusätzlich durch heiße Druckluft und Öl. Für jeden Behandlungsvorgang der Handstücke wird der vorhandene Behälter mit heißem Wasser gefüllt oder beheizt. Nach der Behandlung wird die Flotte abgepumpt.

Bei der bekannten Vorrichtung ist eine gezielte Reinigung Desinfektion der Fluidleitungen im Handstück nicht möglich.

Aus der EP-A-0 494 031 ist eine Flüssigkeitsverteilungsvorrichtung für einen Apparat zum Reinigen, Desinfizieren und Schmieren zahnärztlicher Handstücke beschrieben, wobei das Handstück jeweils auf ein Steckkupplungsteil am Apparat aufsteckbar ist. Durch das Steckkupplungsteil erstrecken sich in nicht klar beschriebener Weise zwei Durchgänge,die mittels durch Ventile gesteuerte Leitungsführungen in komplizierter Anordnung eine Durchführung für Wasser, Desinfektionsmittel, Druckluft und Öl in den sogenannten Antriebskanal und die Sprühkanäle des Handstücks ermöglichen sollen. Dabei sollen das Desinfektionsmittel zugleich durch den Antriebskanal und den Medienkanal des Handstücks führbar sein, während das Öl nur in den Antriebskanal führbar sein soll. Dies soll eine besondere Gestaltung des zugehörigen Steckkupplungsteils gewährleisten, s. letzte Zeile auf S. 5 dieser Druckschrift. Offenbar scheint dies nur durch die Verwendung von jeweils für das Desinfizieren und das Pflegen mit Öl unterschiedliche Steckkupplungsteile möglich zu sein, was die Handhabung natürlich wesentlich erschweren würde.

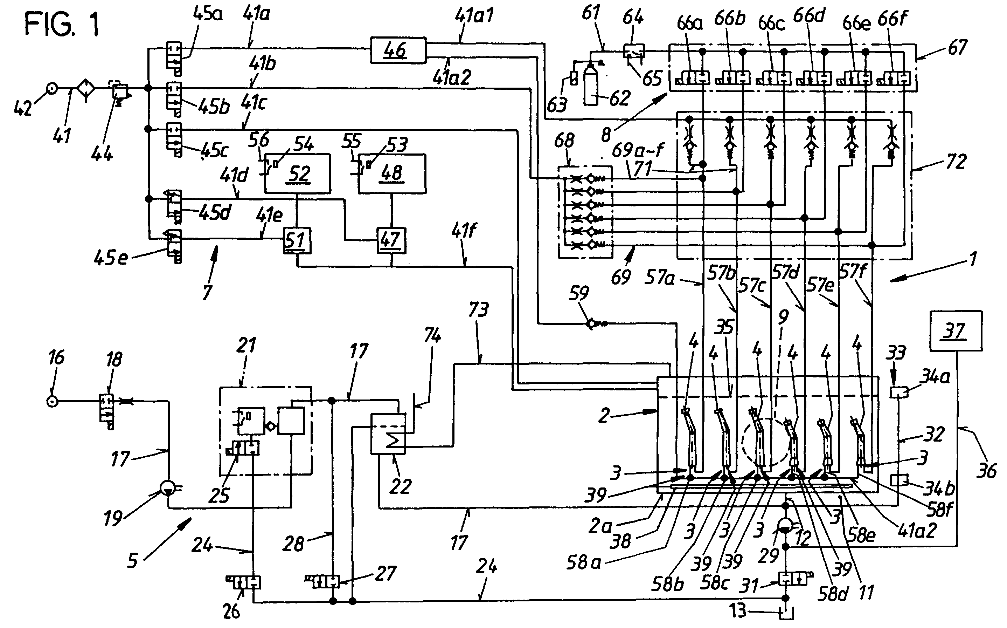

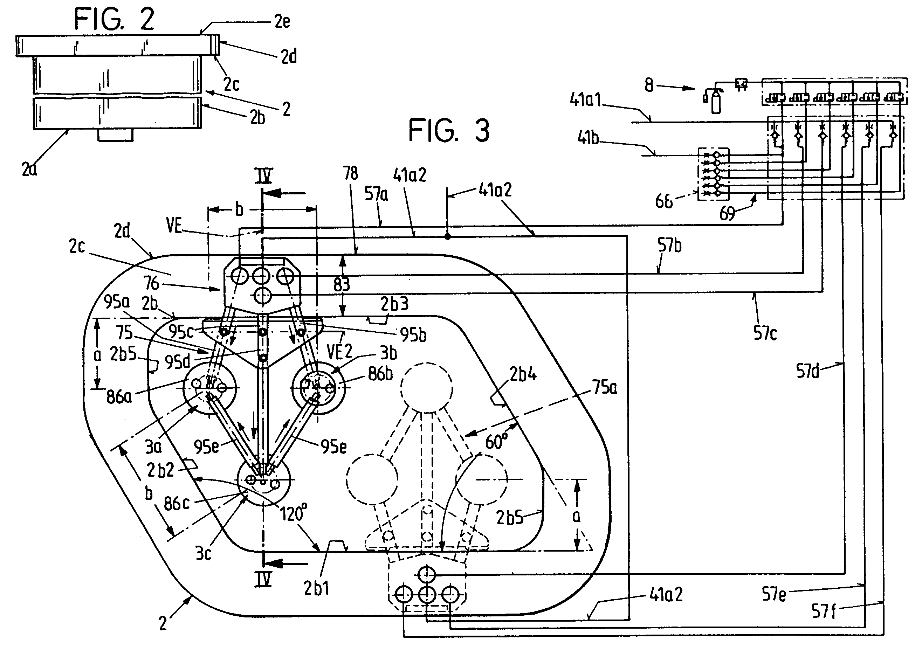

In der WO 88/02621 sind mehrere Ausführungsbeispiele einer Vorrichtung zum Reinigen und Pflegen Von zahnärztlichen Handstücken beschrieben. Bei den Ausführungsbeispielen gemäß Figuren 1, 1a und 2 ist ein hülsenförmiges Zwischenstück vorgesehen, dessen vorderes Ende komplementär zum hinteren Ende eines Handstücks und dessen hinteres Ende komplementär zum vorderen Ende eines Anschlußteiles für das Handstück ausgebildet ist. Im Zwischenstück erstrecken sich Verbindungsleitungen zur Verbindung einer Druckluftleitung im Anschlußteil mit einer oder zwei Medienleitungen im Handstück. Diese Medienleitungen können so mit Druckluft ausgeblasen werden, BeimAusführungsbeispiel gemäß Figur 2 weist das Zwischenstück außerdem einen Verbindungskanal auf zur Verbindung einer seitlich angeschlossenen Öl-Zuführungsleitung mit einem sich im Handstück erstreckenden Druckluftkanal. Diese bekannte Ausgestaltung ermöglicht ein Ausblasen des Fluidkanals und ein Schmieren von Getriebeteilen, zu denen der Druckluftkanal führt.

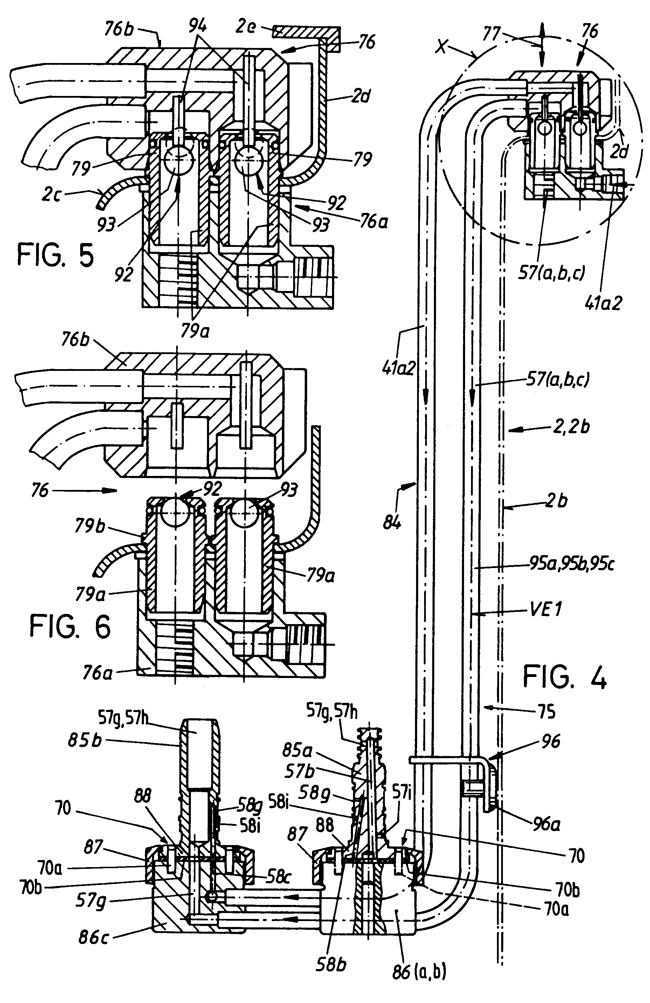

Die Ausführungsbeispiele gemäß Figuren 3 bis 5 von WO 88/02621 zeigen anstelle eines Zwischenstücks eine Ausblashülse mit den Verbindungskanälen des Zwischenstücks vergleichbaren Verbindungskanälen. Die Ausführungsbeispiele gemäß Figuren 3 und 4a sowie 4b ermöglichen ein Schmieren eines zugehörigen Druckluftkanals im Handstück und ein Ausblasen von Medienkanälen im Handstück, wobei die Ausblas-Druckluft durch eine in der Ausblashülse angeordnete und durch den Druck des Schmiermittels (Figur 3) oder von Hand (Figur 4a, 4b) antreibbare Druckluftpumpe erzeugt wird.

Beim Ausführungsbeispiel gemäß Figur 5 von WO 88/02621 sind die Verbindungsleitungen in der Ausblashülse jeweils mit einem zugehörigen unter Druck stehenden Vorratsbehälter verbunden von denen der eine ein Reinigungsgas (Druckluft) und der andere eine Reinigungs- und Schmierflüssigkeit (Öl) enthält.

Figur 6 von WO 88/02621 zeigt ein Ausführungsbeispiel, bei dem eine Ausblashülse und ein besonderes Anschlußteil vorgesehen sind, die die Zuführung von Drukluft und einem Kühlmedium oder einer Kühlflüssigkeit zu den Kanälen des Handstücks ermöglichen. Ein Schmieren bzw. Pflegen von Antriebs- und Verschleißteilen des Handstücks ist bei dieser Ausgestaltung folglich nicht möglich.

Die Figuren 7a und 8 von WO 88/02621 zeigen Ausblashülsen in Pistolenform, die es jeweils ermöglichen, die Medienkanäle eines Turbinenhandstücks mit Druckluft auszublasen und ein flüssiges Schmiermittel in den Antriebskanal unter Druck einzuführen.

Wie bereits bei der Ausgestaltung nach Figur 5 ist auch bei den Ausgestaltungen nach Figur 7a und 8 die Schmierung unbefriedigend, weil nach der Schmierung eine verhältnismäßig große Menge Öl im Antriebskanal verbleibt.

Von den vorher beschriebenen bekannten Ausgestaltungen ist die Ausgestaltung nach Figur 5 von WO 88/02621 von der eingangs angegebenen Art.

Der Erfindung liegt die Aufgabe zugrunde, eine Vorrichtung der eingangs angegebenen Art so auszugestalten, daß eine intensivere Reinigung und/oder Desinfektion und Pflege der Handstücke möglich ist.

Diese Aufgabe wird durch die Merkmale des Anspruchs 1 gelöst.

Bei der erfindungsgemäßen Vorrichtung sind jedem Träger zwei Zuführungsleitungen zugeordnet, von denen die eine Zuführungsleitung mit den die mechanischen Antriebsteile aufnehmenden Hohlräume des Handstücks verbindbar ist und die zweite Zuführungsleitung mit der oder den Fluidleitungen im Handstück verbindbar ist. Dabei ist die Anordnung vorzugsweise so getroffen, daß mit der zuerst genannten Zuführungsleitung wahlweise Druckluft, insbesondere heiße Druckluft, und ein Pflegemittel zuführbar ist, während mit der zweiten Zuführungsleitung lediglich Druckluft, vorzugsweise heiße Druckluft, zuführbar ist. Die erste Zuführungsleitung kann somit der Reinigung, Desinfektion und Pflege dienen, während die zweite Zuführungsleitung der Reinigung und/oder Desinfektion dient. Die erfindungsgemäße Vorrichtung ermöglicht eine vollständige Reinigung, Desinfektion und Pflege des wenigstens einen Handstücks, wobei sich diese Behandlungsvorgänge sicher und mit geringem Zeitaufwand, Handhabungsaufwand und Vorrichtungsaufwand durchführen lassen. Im Rahmen der Erfindung ist es auch möglich, weitere Hohlräume des bzw. der Handstücke zu reinigen, desinfizieren und zu pflegen.

In den Unteransprüchen sind Merkmale enthalten, die zur Problemlösung beitragen, ein halb- oder vollautomatisches Reinigen, Desinfizieren und/oder Pflegen ermöglichen, einfache und kostengünstig herstellbare Ausgestaltungen kleiner und handhabungsfreundlicher Bauweisen ermöglichen und auch eine umweltfreundliche Arbeitsweise sowie Energieeinsparung ermöglichen.

Eine Weiterbildung der Erfindung bezieht sich im weiteren auch darauf, zur Reinigung und/oder Desinfektion des Handstücks zusätzlich eine Beaufschlagung des Handstücks mit Ultraschallwellen auszuüben. Dabei werden vorher wenigstens die die Getriebeteile aufnehmenden Hohlräume des Handstücks und vorzugsweise auch die Fluidkanäle bzw. - leitungen oder auch weitere Hohlräume des Handstücks entwässert, damit bei der Beaufschlagung mit Ultraschallwellen keine Gravitations- und Kavitationsschäden entstehen.

Nachfolgend werden die Erfindung und weitere durch sie erzielbare Vorteile anhand bevorzugter Ausführungsbeispiele und einer Zeichnung näher erläutert. Es zeigt
- Fig. 1: eine erfindungsgemäße Vorrichtung zum Reinigen, Desinfizieren und/oder Pflegen von Handstücken als Pflegeplatz in schematischer Darstellung;
- Fig. 2: einen Behälter der Vorrichtung in der Seitenansicht;
- Fig. 3: den Behälter mit Trägereinheiten für die Handstücke in schematischer Draufsicht und in vergrößerter Darstellung;
- Fig. 4: den Teilschnitt IV-IV in Fig. 3;
- Fig. 5: eine in Fig. 4 mit X gekennzeichnete Steckverbindung in vergrößerter Darstellung;
- Fig. 6: die Steckverbindung in geöffneter Stellung;

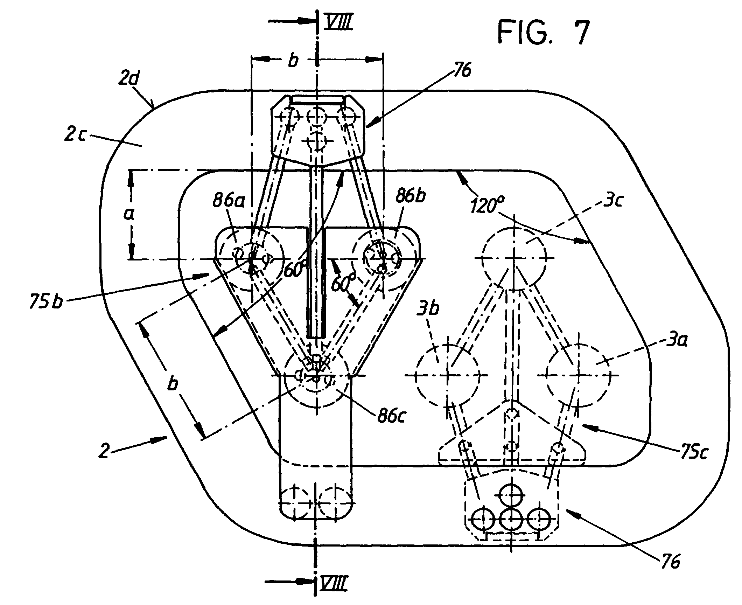

- Fig. 7: den Behälter in der Draufsicht mit Trägereinheiten abgewandelter Ausgestaltung;

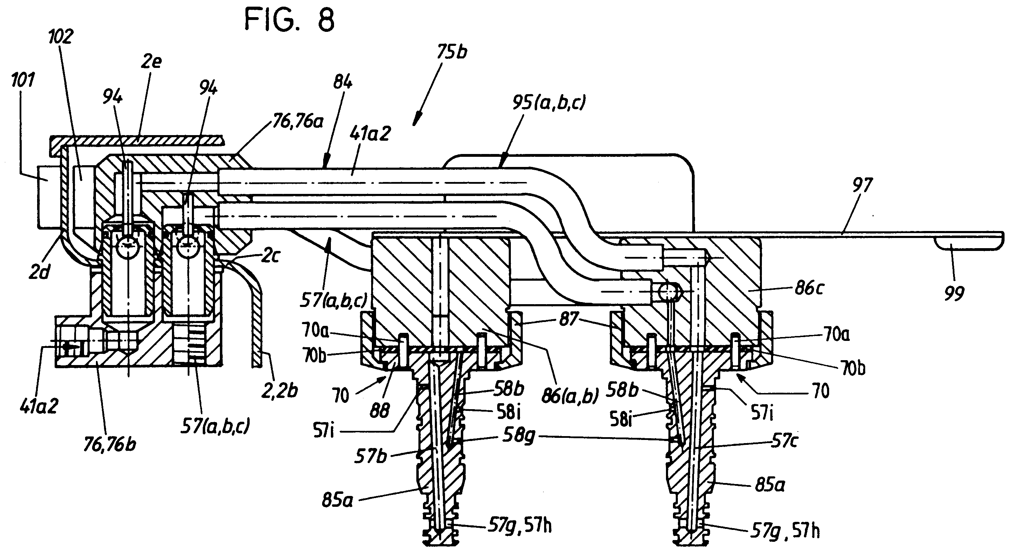

- Fig. 8: den Teilschnitt VIII-VIII in Fig. 7;

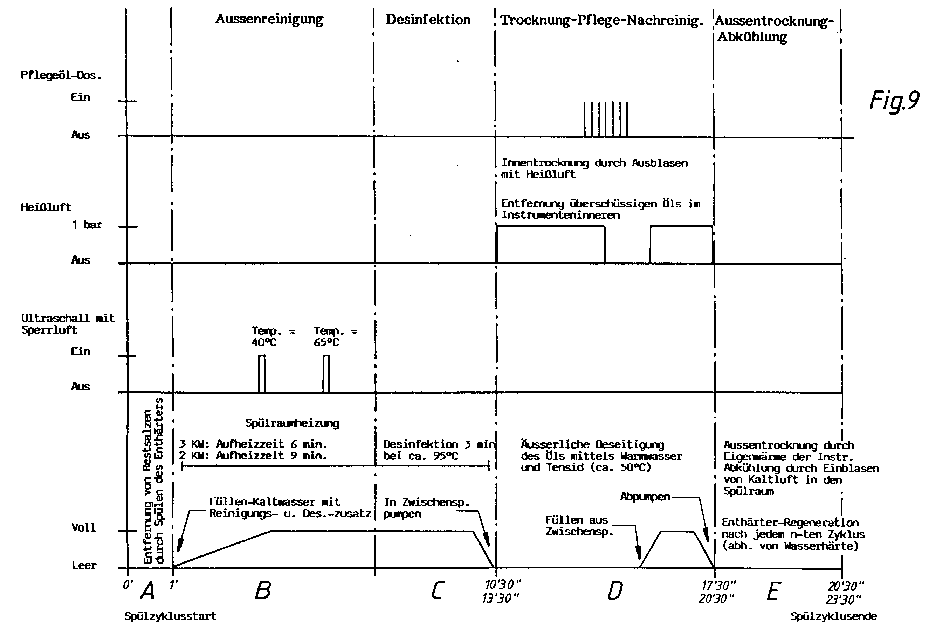

- Fig. 9: ein Funktionsschema der Vorrichtung.

Die Hauptteile der Vorrichtung 1 sind ein topfförmiger Spülbehälter 2 mit einer Mehrzahl Halterungen 3 im Innenraum des Behälters 2 für jeweils ein Handstück 4, beim vorliegenden Ausführungsbeispiel sechs Halterungen 3, ein Wasserversorgungssystem 5, mit dem Wasser dem Spülbehälter 2 zugeführt als auch von ihm angeführt werden kann, ein Druckluftversorgungssystem 7, ein Pflegemittelversorgungssystem 8 und eine Ultraschallreinigungseinrichtung, von der nur ein Ultraschallschwinger 9 zur Beaufschlagung des Spülbehälters 2 mit Ultraschall dargestellt ist.

Der Spülbehälter 2 weist im Bereich seines Bodens 11 eine Zu- und Abführungsleitung 12 auf, die Teil des Wasserversorgungssystems 5 ist , und mit einem Wasserabfluß 13. In der an einen Wasseranschluß 16 anschließbaren Wasserzuführungsleitung 17 sind in Strömungsrichtung hintereinanderliegend angeordnet: ein erstes Elektromagnetventil 18, eine Pumpe 19, insbesondere zur Druckerhöhung, eine Enthärtereinrichtung 21 und ein Kondensator 22. Die Enthätereinrichtung 21 ist durch eine Abführungsleitung 24, in der ein oder zwei Elektromagnetventile 25, 26 hintereinanderliegend angeordnet sind, direkt mit dem Wasserabfluß 13 verbunden, um für eine Spülung und Regeneration der Enthärtereinrichtung das Abwasser unmittelbar in den Wasserabfluß 13 abführen zu können. Ein viertes Elektromagnetventil 27 ist in einem die Wasserzuführungsleitung 17 hinter der Enthärtereinrichtung 21 und die Abführungsleitung 24 hinter dem Elektromagnetventil 26 miteinander verbindenden Leitungsabschnitt 28 angeordnet. In der Zu- und Abführungsleitung 12 sind in Richtung des Wasserabflusses 13 eine zweite Pumpe 29 und ein fünftes Elektromagnetventil 31 hintereinanderliegend angeordnet. Die Wasserzuführungsleitung 17 ist zwischen dem Spülbehälter 2 und der zweiten Pumpe 29 mit der Zu- und Abführungsleitung 12 verbunden, wobei in diesem Bereich auch ein weiterer Leitungsabschnitt 32, der zu einer Wasserniveau-Anzeigeeinrichtung oder Meßeinrichtung 33 mit einem Niveauschalter 34a, 34b führt. Der maximale Wasserfüllstand im Spülbehälter 2 ist mit 35 bezeichnet. Er befindet sich oberhalb der in oder auf die Halterungen 3 gesetzten Handstücke 4. Zwischen der zweiten Pumpe 29 und dem Elektromagnetventil 31 zweigt eine weitere Zweigleitung 36 ab, die zu einem Zwischenspeicher 37 führt, der oberhalb des Spülbehälters 2 angeordnet ist. Im Bodenbereich des Spülbehälters 2 ist eine elektrische Heizvorrichtung 38 mit wenigstens einem elektrischen Heizelement angeordnet.

Jede Halterung 3 weist vorzugsweise einen runden Kupplungszapfen auf, auf den das zugehörige Handstück 4 mit einer entsprechend runden Kupplungsausnehmung wahlweise aufsteckbar und abziehbar ist. hierdurch wird jeweils eine Steck/Dreh-Kupplung 39 gebildet, wie sie zur drehbaren Verbindung von Handstücken 4 mit zugehörigen Versorgungsteilen (nicht dargestellt) bekannt sind.

Das Druckluftversorgungssystem 7 weist fünf parallel geschaltete Druckluftleitungen 41a bis 41e auf, die von einer gemeinsamen Druckluftversorgungsleitung 41 ausgehen, die an eine Druckluftquelle 42 anschließbar ist und in der ein Luftfilter und ein Druckregelventil 44 angeordnet sind. In jeder Druckluftleitung 41a-41e ist ein Elektromagnetventil 45a-45e angeordnet. Außerdem ist in der Druckluftleitung 41a ein vorzugsweise elektrisch betriebener Lufterhitzer 46 angeordnet, in dem oder hinter dem die Druckluftleitung 41a in zwei Druckluftleitungszweige 41a1 und 41a2 verzweigt. Die Druckluftleitung 41c mündet in den Spülbehälter 2 oberhalb des maximalen Wasserfüllstandes 35. In der Druckluftleitung 41d ist eine Reinigungsmittel-Dosiervorrichtung 47 angeordnet, die direkt oder durch einen Leitungsabschnitt mit einem Reinigungsmittelbehälter 48 verbunden ist. In der Druckluftleitung 41e ist eine Tensid-Dosiervorrichtung 51 angeordnet, die direkt oder durch einen Leitungsabschnitt mit einem Tensid-Vorratsbehälter 52 verbunden ist. Beiden Vorratsbehältern 48, 52 ist jeweils ein Füllstandsmesser 53, 54 und ein elektrischer Schalter 55, 56 zugeordnet. Hinter den Dosiervorrichtungen 47, 51 sind die Druckluftleitungen 41d und 41e zu einer gemeinsamen Druckluftleitung 41f zusammengeführt, die ebenfalls in den Spülbehälter 2 oberhalb des maximalen Wasserfüllstandes 35 mündet.

Jeder Halterung 3 sind zwei Zuführungsleitungen, nämlich eine erste Zuführungsleitung 57a-57f und eine zweite Zuführungsleitung 58a-58f zugeordnet. Die Zuführungsleitungen 58a-58f sind jeweils mit dem gemeinsamen Zuführungsleitungszweig 41a2 verbunden, in dem ein in Strömungsrichtung öffnende Rückschlagventil 59 angeordnet ist. Die ersten Zuführungsleitungen 57a-57f sind mit einer gemeinsamen Pflegemittel-Versorgungsleitung 61 verbunden, die von einem Pflegemittel-Druckbehälter 62 ausgeht, z.B. eine Sprayflasche, wobei ein elektrisch ansteuerbares Ventil 63 vorgesehen ist, mit dem die Abgabe des Pflegemittels in die Versorgungsleitung 61 durch Öffnen und Schließen wahlweise steuerbar ist. Außerdem ist in der Versorgungsleitung 61 eine Meß- oder Überwachungsvorrichtung 64 mit einem elektrischen Schalter 65 angeordnet. In den ersten Zuführungsleitungen 57a-57f ist jeweils ein Elektromagnetventil 66a bis 66f angeordnet, die in einem Pflegemittelverteiler 67, z.B. in Form eines Ventilblocks zusammengefaßt sind.

Die Druckluftleitung 41b verzweigt in einem Druckluftverteiler 68 in der Anzahl der Halterungen 3 entsprechende Anzahl Druckluftleitungszweige 69a-69f, in denen jeweils eine Drossel und ein in Strömungsrichtung öffnendes Rückschlagventil angeordnet sind, und die jeweils mit einer der ersten Zuführungsleitungen 57a-57f verbunden sind. Die ersten Zuführungsleitungen 57a-57f sind außerdem jeweils durch einen Leitungsabschnitt 71 mit dem Druckluftleitungszweig 41a1 verbunden, wobei in dem Leitungsabschnitt 71 jeweils ebenfalls eine Drossel und ein in Strömungsrichtung öffnendes Rückschlagventil angeordnet sind. Die vorbeschriebenen Leitungsverbindungen sind einem Druckluftverteiler 72 zugeordnet.

Vom Bereich oberhalb des maximalen Wasserfüllstandes 35 erstreckt sich eine Dampfleitung 73 vom Spülbehälter 2 zum Kondensator 22, in dem aus dem Spülbehälter 2 strömender Dampf kondensiert und dem Wasserversorgungssystem 5 wieder zugeführt wird. Die Dampfleitung 73 endet im Bereich des Kondensators 22 als eine freie Abluftleitung 74.

Wie aus den Fig. 3 bis 7 zu entnehmen ist, sind mehrere, hier jeweils drei Halterungen 3 zu einer Handstück-Trägereinheit 75 zusammengefaßt, die in einer Steckfassung 76 lösbar mit dem Spülbehälter 2 verbunden ist, wobei die zugehörigen Zuführungsleitungen 57a-57f und 41a2 die Steckfassung 76 dicht durchsetzen. Auf diese Weise sind verschiedene, an vorgegebene Handstückkonstruktionen angepaßte Halterungen 3 handhabungsfreundlich und schnell montierbar bzw. demontierbar. Die Vorrichtung 1 kann somit in einfacher Weise und schnell durch Einbau von vorhandenen passenden Trägereinheiten 75 an Handstücke 3 unterschiedlicher Kupplungsgrößen und - formen umgerüstet werden.

Die Steckfassung 76 ist vorzugsweise am oberen Rand des Spülbehälters 2 nach außen versetzt angeordnet. In dieser Position läßt sich vorteilhaft eine vertikale Steckrichtung (Pfeil 77) realisieren, so daß beim Einstecken der Trägereinheit 75 in den Spülbehälter 2 sich die Steckfassung 76 raumsparend verbinden läßt. Die Steckfassung 76 besteht aus einem am Spülbehälter 2 befestigten Steckfassungsteil 76a und einem an der Trägereinheit 75 befestigten Steckfassungsteil 76b. Das Steckfassungsteil 76a kann aus zwei Teilen bestehen, die zu beiden Seiten der Behälterwand 2b des Spülbehälters 2 angeordnet sind, wobei das eine Teil, hier das innere Teil, die Behälterwand 2b in einem passenden Loch durchsetzt und mit einer Schulter am inneren Lochrand anliegt, während das andere, hier äußere, Teil von der anderen Seite der Behälterwand 2b her muffenförmig auf das eine Teil aufgesetzt und damit verbunden ist, z.B. lösbar durch Gewinde oder unlösbar z.B. durch Schweißen oder Löten.

Vorzugsweise ist der Spülbehälter 2 in seinem oberen Randbereich mit einer von der Behälterwand 2b ausgehenden horizontalen Flanschwand 2c verbreitert, an die sich nach oben eine etwa vertikale Randwand 2d anschließt. Die seitliche Verbreiterung ist größer bemessen als das Steckfassungsteil 76a, so daß diese im Bereich der so gebildeten Randverbreiterung 83 angeordnet und Flanschwand 2c durchfassen kann. Mit 2e ist ein Deckel bezeichnet, der auf der Randwand 2d aufliegt und somit die Trägereinheit 75 abdeckt.

Das obere Teil des Steckfassungsteils 76a ragt zapfenförmig nach oben vor und bildet somit einen oder mehrere, vorzugsweise in der Anzahl der die Steckfassung 76 durchsetzenden Zuführungsleitungen 57a-57c und 41a2 vorhandene Zapfen 79, auf die das dem Trägereinheit 75 zugeordnete Steckfassungsteil 76b mit einer oder mehreren Steckausnehmung entsprechender Anzahl und entsprechender Form und Größe schließend aufsteckbar ist. Die Zapfen 79 sind vorzugsweise durch jeweils eine Schulter 79b aufweisende Hülsen 79a gebildet, die in das Steckfassungsteil 76a eingesetzt und befestigt sind.

Das dem Träger 75 zugeordnete Steckfassungsteil 76b ist durch einen vorzugsweise Z-förmig geformten Schaft 84 mit mehreren, hier drei Halterungen 3 verbunden, die in den Ecken eines gedachten Dreiecks angeordnet sind, wobei zwei der Behälterwand 2b nahegelegene Halterungen 3a, 3b symmetrisch zu einer sich rechtwinklig zur Umfangswand 78 erstreckende Vertikalebene VE angeordnet sind und eine zugehörige weitere Halterung 3c zur dem zugehörigen Steckfassungsteil 76b abgewandten Seite hin versetzt ist, wobei die Halterungen 3a, 3b, 3c auf den Eckpunkten des gedachten gleichseitigen Dreiecks angeordnet sind.

Wie bereits erwähnt, sind die Halterungen 3 durch runde Steckzapfen 85 gebildet, die sich bei der stehenden Anordnung der Handstücke 4 beim Ausführungsbeispiel gemäß Fig. 1 bis 5, von einem jeder Halterung 3 zugeordneten Trägerbasisteil 86a, 86b, 86c nach oben erstrecken und vorzugsweise lösbar befestigt sind. Hierzu kann eine Überwurfmutter 87 dienen, die einen Basisflansch 88 des zugehörigen Steckzapfens 85 übergreift und auf das zugehörige, vorzugsweise runde Trägerbasisteil 86 aufgeschraubt ist. Um zu gewährleisten, daß die im jeweiligen Trägerbasisteil 86a, 86b, 86c vorhandenen Kanalabschnitte 57a-57g und 58a - 58f mit den zugehörigen Kanalabschnitten 57a-57g und 58a-58f im zugehörigen Steckzapfen 85a-85c miteinander fluchten, ist eine Positioniervorrichtung 70 zwischen dem Trägerbasisteil 86a, 86b, 86c und dem Steckzapfen 85a-85c vorgesehen, die diese Teile in der bestimmten Stellung zueinander positioniert und vorzugsweise durch eine Steckverbindung gebildet ist. Bei der vorliegenden Ausgestaltung wird die Positioniervorrichtung 70 durch einen oder zwei Zentrierstifte 70a gebildet, die jeweils in Löchern im Trägerbasisteil 86a, 86b, 86c fest eingesetzt sind und in den Basisflansch 88 mit geringem Bewegungsspiel einfassen, so daß letzterer angesteckt werden kann. Die vorhandene Zentrierung zwischen dem Basisflansch 88 und der Überwurfmutter 87 kann ebenfalls zur Positionierung bzw. Arretierung des Basisflansches beitragen.

Zur Abdichtung der Kanalabschnitte zwischen dem Trägerbasisteil 86a, 86b, 86c und dem Basisflansch 88 sind Dichtungen vorzusehen. Bei der vorliegenden Ausgestaltung ist eine gemeinsame Dichtung in Form einer zwischen dem Trägerbasisteil 86a, 86b, 86c und Basisflansch 88 angeordneten Dichtungsscheibe 70b vorgesehen, die Löcher zur Aufnahme der Zentrierstifte und für die Kanäle aufweist.

Diese Ausgestaltung ermöglicht es, unterschiedliche Handstücke 4, und zwar unterschiedliche Handsrücke 4 eines Herstellers und/oder eines oder mehrerer verschiedener Hersteller (Fremdhersteller) am Träger 75 anzuordnen und behandeln zu können. Die diesbezüglich gleich ausgebildeten Basisflansche 88 bilden somit Adapter für die Anordnung verschiedener Steckzapfen 85.

Der Steck/Dreh-Kupplung 39 sind Verrastungselemente zugeordnet, die eine lösbare Verrastung der Handstücke 4 in der aufgesteckten Position bewirken, wie es bei Handstücken für eine Schnellverbindung üblich ist.

Für mittels eines Luftmotors luftbetriebene Behandlungsinstrumente und für durch einen elektrischen Motor elektrisch betriebene Behandlungsinstrumente werden in der Praxis Steck/Dreh-Kupplungen 39 mit Steckzapfen 85 unterschiedlicher Form und/oder Größe hergestellt. Bei der vorliegenden Ausgestaltung sind die mit 85a bezeichneten Steckzapfen für ein druckluftbetriebenes Handstück 4 konzipiert, wobei der mit 85b bezeichnete Steckzapfen für ein elektrisch angetriebenes Handstück 4 konzipiert ist.

Die Druckluftleitung 41a2 ist an das Steckfassungsteil 76a angeschlossen, und erstreckt sich durch die Steckfassung 76 und den Schaft 84 bis zu den Trägerbasisteilen 86a, 86b, 86c. Darin zweigt jeweils die zugehörige zweite Zuführungsleitung 58a bis 58c ab und erstreckt sich als achsparalleler Kanal durch den zugehörigen Steckzapfen 85, aus dem sie in eine Mündungsöffnung 58g radial ausmündet, vorzugsweise in einer Umfangsnut und zwischen zwei Ringdichtungen, wie es an sich bei Steck/Dreh-Kupplungen bekannt ist.

Die ersten Zuführungsleitungen 57a-57c sind ebenfalls an das dem Spülbehälter 2 zugeordnete Steckfassungsteil 76a angeschlossen, und sie erstrecken sich als separate Zuführungskanäle 57g im Trägerteil 75 weiter, wobei sie sich im Bereich des zugehörigen Steckzapfens 85 koaxial erstrecken und von den Mündungsöffnungen 58g axial versetzt an Mündungsöffnungen 57h aus dem zugehörigen Steckzapfen 85 austreten, und zwar koaxial bei einem Steckzapfen 85b für ein elektromotorisch betriebenes Handstück und ebenfalls radial in einer Umfangsnut zwischen zwei Dichtungsringen bei einem Steckzapfen 85a für ein sog. Turbinen-Handstück. Den Mündungsöffnungen 57h, 58g der Zuführungskanäle 57a-57c gegenüberliegend sind im jeweils zugehörigen Handstück 4 Fluidkanäle oder die Hohlräume weitergeführt, die den mechanischen Getriebezug, z.B. eine Antriebsspindel, aufnehmen.

In dem Steckfassungsteil 76a ist in jedem ersten Zuführungskanal 57a-57c und in der Druckluftleitung 41a2 ein Sperrventil 92 vorgesehen, dessen Zweck es ist, den zugehörigen Kanal dann zu verschließen, wenn keine Trägereinheit 75 montiert wird. Diese Sperrventile 92 sind jeweils durch eine Ventilkugel 93 in einem verbreiterten Kanalabschnitt im Steckfassungsteil 76a gebildet, der in Strömungsrichtung ein eine Ventilöffnung umgebender Ventilsitz vorgeordnet ist. Wenn keine Trägereinheit 75 vorhanden ist, schließen die Ventilkugeln 93 die Sperrventile 92 aufgrund eines vorhandenen pneumatischen Schließdruckes, wobei die Ventilkugeln 93 gegen den zugehörigen Ventilsitz drückende Druckfedern vorhanden sein können. Beim Vorhandensein einer montierten Trägereinheit 75 werden die Ventilkugeln 93 durch Stifte 94 im aufgedrückten Zustand gehalten, die im Steckfassungsteil 76b koaxial zum Sperrventil 92 befestigt sind und so lang bemessen sind, daß sie die Ventilöffnung bei Gewährleistung eines Ringspaltes durchfassen und die Ventilkugeln 93 von den Ventilsitzen abheben. Der oder die Zapfen 79 sind durch sie umgebende Ringdichtungen zwecks Abdichtung abgedichtet.

Bei der vorliegenden Ausgestaltung besteht der Schaft 84 der Trägereinheit 75 aus die Zuführungsleitungskanäle 57a-57c und die Druckluftleitung 41a2 bildenden vier dünnen Rohren 95a-95d, vorzugsweise aus Metall, die sich z-förmig zwischen dem Steckfassungsteil 76b und den drei Trägerbasisteilen 86 erstrecken und damit vorzugsweise seitlich dicht verbunden sind, z.B. durch Kleben oder Löten. Die vertikalen Abschnitte der die Zuführungsleitungen 57a-57c bildenden Rohre 95a-95c erstrecken sich in einer rechtwinklig zur Vertikalebene VE verlaufenden Vertikalebene VE2 nahe der Behälterwand 2b, und sie sind jeweils zum zugehörigen Trägerbasisteil 86 abgebogen. Das die Druckluftleitung 41a2 bildende Rohr 95d erstreckt sich in der Vertikalebene VE etwa parallel zum mittleren Rohr 95b zum Trägerbasisteil 86c. Von hier aus ist die Druckluftleitung 41a2 durch zwei V-förmig angeordnete horizontale Rohrstück 95e mit den Trägerbasisteilen 86a, 86b verbunden. Durch die Anordnung der Rohre 95 in mehreren Ebenen ergibt sich eine stabile Ausgestaltung.

Im unteren Bereich der Rohre 95 ist außenseitig ein vorzugsweise winkelförmiges Stützteil 96 angeordnet, das nicht nur die Rohre 95a, 95b, 95c miteinander verbindet und somit stabilisiert, sondern auch eine seitliche Abstützung an der Innenwand des Spülbehälters 2 gewährleistet. Vorzugsweise ist der äußere vertikale Schenkel des Stützteils 96 mit einem durchgehenden oder zwei voneinander beabstandeten Pufferstücken 96a aus weichem Material besetzt, die eine weiche Abstützung in an der Umfangswand 2b sichern.

Es ist eine weitere Trägereinheit 75a gleicher Ausgestaltung jedoch in um 180° horizontal verdrehter Position vorgesehen, wobei diese Trägereinheit 75a in zur Trägereinheit 75 rechtwinklig zur Vertikalebene VE versetzter Position auf dem gegenüberliegenden Randflansch 2c mit dem Steckfassungsteil 76b in jener Steckfassung 76 aufliegt. Aufgrund der verdrehten Anordnung der Trägereinheiten 75, 75a nehmen die sechs Stück vorhandenen Halterungen 3 bzw. Trägerbasisteile 86 die Form eines Parallelogramms ein. Vorzugsweise ist die horizontale Ouerschnittsform des Spülbehälters 2 an diese Form angepaßt und somit parallelogrammförmig, wobei die Ecken dieser Parallelogrammform vorzugsweise gerundet sind.

Zur Trägereinheit 75a erstrecken sich in entsprechender Weise die ersten Zuführungsleitungen 57d-57f und eine Zweigleitung des Druckluftleitungszweiges 41a2.

Bei der vorliegenden Ausgestaltung schließen die jeweils eine Innenecke begrenzenden ebenen und vertikalen Behälterseitenwände 2b1 bis 2b4 jeweils einen Winkel von etwa 60° oder etwa 120° ein. Dabei können im Bereich der spitzwinkligen Innenecken jeweils der Anfangsbereich a der Behälterseitenwände 2b2, 2b4, die sich bezüglich der Trägereinheit 75 schräg erstrecken oder der Vertikalebene VE gegenüberliegen, zu letzterer parallel angeordnet sein. Diese Behälterseitenwandteile sind jeweils mit 2b5 bezeichnet. Die Breite a dieser Behälterseitenwandteile 2b2, 2b4 entspricht etwa dem Abstand, den die Halterungen 3a, 3b von der Behälterseitenwand 2b3 aufweisen.

Ein Vorteil der schiefwinkligen bzw. parallelogrammförmigen Ouerschnittsform des Spülbehälters 2 besteht in einer guten Raumausnutzung bzw. Anpassung an die Trägereinheiten 75, 75a, so daß möglichst wenig Spülwasser verbraucht wird und trotzdem die eingesetzten Handstücke 4 satt umspült werden.

Ein weiterer Vorteil besteht darin, daß diese Form die Wirksamkeit der Ultraschallreinigung verbessert. Aufgrund der schiefwinkligen bzw. parallelogrammförmigen Form werden an der Behälterwand 2b des Spülbehälters 2 durch Reflexion erzeugte solche Reflexionsschallwellen weitgehend vermieden, die einander direkt entgegengesetzt sind und dadurch deren Leistungsfähigkeit beeinträchtigen würden. Wie insbesondere aus Fig. 3 zu entnehmen ist, sind die Halterungen 3 und die aufgesteckten Handstücke 4 der gegenüberliegenden Behälterwand 2b vollflächig zugewandt und somit durch die davon ausgehenden Ultraschallwellen ohne wesentliche Wellenschattenbildung und ohne wesentlichen Leistungsverlust wirksam Eventuell schädigende Resonanzschwingungen, Schwingungsankopplungen oder Schwingungseinleitungen von außen in das Handstückinnere werden weitgehend vermieden. Bei der vorliegenden Ausgestaltung ist der Ultraschallschwinger 9 in mittlerer Höhe des Spülbehälters 2 und im mittleren Bereich einer Seitenfläche an der Außenseite der Behälterwand 2b befestigt, z.B. durch Kleben.

Die Ausgestaltung nach den Fig. 7 und 8, bei der gleiche oder vergleichbare Teile mit gleichen Bezugszeichen versehen sind, unterscheidet sich von der vorbeschriebenen lediglich dadurch, daß anstatt einer stehenden Anordnung für die Handstücke 4 eine hängende Anordnung vorgesehen ist, d.h., die Trägerbasisteile 86 befinden sich mit den Steckzapfen 85 in um 180° verdrehter Position im oberen Bereich des Spülbehälters 2, wobei die Steckzapfen 85 nach unten weisen und die Handstücke 4 hängend von unten aufgesteckt sind. Zur Stabilisierung dieser Anordnung ist ein horizontaler Stützarm 97 vorgesehen, der sich vom dem Steckfassungsteil 76b am weitesten entfernten Trägerbasisteil 86c zum gegenüberliegenden Rand des Spülbehälters 2 erstreckt und mit einem an seiner Unterseite befestigten Dämpferteil 99 aus weichem Material auf dem gegenüberliegenden Randflansch 2d aufliegt. Bei dieser Ausgestaltung erstrecken sich der Schaft 84 und die Rohre 95a-95c im wesentlichen horizontal. Im übrigen ist diese Trägereinheit 75b und auch die weitere entsprechend ausgebildete, jedoch verdreht angeordnete Trägereinheit 75c entsprechend der Trägereinheit 75 bzw. 75a ausgebildet bzw. angeordnet.

Die Träger oder Trägereinheiten 75, 75a, 75b, 75c bilden mit ihren gleich ausgebildeten Anschlußteilen oder vorzugsweise Steckfassungsteilen 76a Adapter, mit denen unterschiedliche Handstücke 4 eines Herstellers und/oder eines oder mehrerer verschiedener Hersteller (Fremdhersteller) am bzw. im Spülbehälter 2 lösbar gehalten und gepflegt werden können.

Bei den vorbeschriebenen Elektromagnetventilen handelt es sich vorzugsweise um 2/2-Wegeventile, die in der einen Schaltstellung den Durchgang der zugehörigen Leitung schließen und in der anderen Schaltstellung öffnen.

Im folgenden wird die Funktion der Vorrichtung 1 anhand der Fig. 9 näher beschrieben, auf deren Abszisse die Zeit und Behandlungsschritte A-E aufgetragen sind und auf deren Ordinate Zeitschritte aufgetragen sind.

Eine Spülung der Enthärtereinrichtung 21 wird beim Behandlungsschritt A nur dann aktiviert, wenn zum Ende des vorausgegangenen Pflegezykluses das Harz der Enthärtereinrichtung 21 mit Sole regeneriert wurde. Hierbei wird die Sole, die bis zum Start eines neuen Pflegezykluses im Hartbehälter verbleibt, mit Frischwasser ausgespült.

Vor einem Pflegevorgang werden die zu pflegenden Handstücke 4 den zugehörigen Haltern 3 durch Aufstecken zugeordnet. Danach wird der Pflegevorgang eingeleitet, der mittels einer nicht dargestellten elektrischen Steuereinrichtung, die durch Signal- und Steuerleitungen mit den zugehörigen Funktionselementen verbunden ist, halb- oder auch vollautomatisch ablaufen kann. Zunächst wird durch Öffnen des Elektromagnetventils 18 mit einem zugeordneten Durchfluß-Mengenbegrenzer der Spülbehälter 2 mit Wasser gefüllt. Nach Erreichen eines minimalen Füllstandes, z.B. dann, wenn die elektrische Heizvorrichtung 38 mit Wasser bedeckt ist, gibt der Niveauschalter 34 ein Signal zum Einschalten der Heizvorrichtung 38 ab. Die Dosierung des Reinigungsmittels erfolgt durch Öffnen des Elektromagnetventils 45d für eine bestimmte Zeitspanne. Dabei wird die dosierte Reinigungsmittelmenge mittels Druckluft durch die Leitung 41f in den Spülbehälter 2 gedrückt. Das Spülwasser strömt durch die zwischen den Steckzapfen 85 und den Instrumenten bzw. Handstücken 4 vorhandene Ringspalte in deren Innenräume (Medienkanäle, Hohlräume für den Werkzeugantrieb), wenn keine Dichtungsringe auf den Steckzapfen jeweils vor und hinter den Mündungsöffnungen 57g, 58g angeordnet sind, und die in den Handstücken 4 befindliche Luft vermag an Öffnungen insbesondere an den vorderen Enden der Handstücke 4 auszuströmen bei stehender Anordnung oder umgekehrt bei hängender Anordnung der Handstücke 4 (Fig. 4 und 8). Wenn die Steckzapfen 85 mit Dichtungsringen bestückt sind, sind Bypaßkanäle 57i, 58i vorzusehen, in die die Zuführungskanäle 57, 58 im Bereich, insbesondere Fußbereich, der Steckzapfen 85 radial nach außen ausmünden und zwar außerhalb des zugehörigen Dichtungsringpaares. Nach dem Abstellen der Wasserzufuhr beim Erreichen des Wasserfüllstandes 35 und dann, wenn das aufgeheizte Wasser eine Temperatur von etwa 40°C erreicht, was durch einen nicht dargestellten Temperaturmesser ermittelt wird, wird das Elektromagnetventil 45a oder 45b geöffnet, wobei Druckluft durch die Zuführungsleitungen 57a-57f in die die mechanischen Antriebselemente aufnehmenden Hohlräume und die Fluidleitungen der Handstücke 4 gleichzeitig eingeblasen und diese Hohlräume ausgeblasen und entwässert werden. Dies kann mit kalter Druckluft oder auch mit heißer, durch den Lufterhitzer 46 erhitzter Druckluft erfolgen. Der Drucklufterhitzer 46 ist hierzu wahlweise einschaltbar bzw. gesteuert. Die die Hohlräume entwässernde Druckluft ist zugleich eine "Sperrluft", die das Wasser darin hindert, in die Hohlräume einzudringen. Die Drosseln in den Druckluftverteilern 72 und auch 68 dienen der gleichmäßigen Verteilung der Druckluft auf alle Zuführungsleitungen 57a-57f.

Wenige Sekunden nach dem Öffnen des Elektromagnetventils 45a wird der oder werden die vorhandenen Ultraschallschwinger 9 für eine kurze Zeit zwecks Ultraschallreinigung erregt. Danach wird diese Druckluftbeaufschlagung abgeschaltet. Bei etwa 65°C wird der gleiche Vorgang wiederholt.

Die vorbeschriebene Druckluftbeaufschlagung der die mechanischen Antriebselemente aufnehmenden Hohlräume ist von wesentlicher Bedeutung. Sie führt zu einer Entwässerung der Hohlräume, so daß bei der nachfolgenden Ultraschallreinigung keine Kavitationsschäden an den Hohlraumwänden und den Oberflächen der Antriebselemente entstehen.

Danach wird das Wasser weiter aufgeheizt bis es kocht bzw. eine Temperatur erreicht, die für eine Desinfektion erforderlich ist. Bei dieser Desinfektion beim Behandlungsschritt C werden die Handstücke innen und außen mit kochendem Wasser benetzt und somit desinfiziert. Dieser Desinfektionsvorgang dauert eine vorbestimmte Zeit. Danach wird das Wasser für eine spätere Wiederverwendung bei einer Nachreinigung mittels der Pumpe 29 in den Zwischerspeicher 37 gepumpt.

Es folgt nunmehr ein Trocknungs- und Pflegevorgang beim Behandlungsschritt D. In dieser Phase werden die Handstücke 4 durch Öffnen des Elektromagnetventils 45b mit durch den Lufterhitzer 46 erhitzter Druckluft ausgeblasen, wobei diese heiße Druckluft durch die Druckluftleitungszweige 41a1 und 41a2 sowohl den Zuführungsleitungen 57a-57f als auch den Zuführungsleitungen 58a-58f gleichzeitig zugeführt wird. Anschließend erfolgt die Pflege durch Öffnen der Ventile 63 und 66a-66f, wodurch das Pflegemittel, hier ein Pflegespray, unter Druck durch die Zuführungsleitungen 57a-57f in die die mechanischen Antriebselemente aufnehmenden Hohlräume der Handstücke 4 eingesprüht wird. Nach diesem Arbeitsschritt, der nur eine kurze Zeitspanne beträgt, werden die vorgenannten Ventile wieder geschlossen, und es wird das Elektromagnetventil 45a oder 45b wieder geöffnet oder das Elektromagnetventil 45a offengelassen, wodurch heiße oder kalte Druckluft durch die Zuführungsleitungen 57a-57f in die die mechanischen Antriebselemente aufnehmenden Hohlräume eingeblasen wird und diese Hohlräume von überschüssigem Pflegemittel ausbläst. Das an den vorderen Enden der Handstücke 4 austretende Öl wird bei einer Nachreinigung mittels aus dem Zwischenspeicher 37 in die Spülkammer 2 zurückgeführten Wasser und einem Tensidzusatz, der kurzzeitig durch Öffnen des Elektromagnetventils 45e in das Wasserbad eingeführt wird, abgereinigt. Während dieser Außenreinigung wird heiße oder kalte Druckluft in die Handstücke 4 eingeführt, so daß kein Wasser eindringen kann, siehe die beiden Heißluft-Zuführungsschritte (mit dünnen Vollinien verdeutlicht) in Fig. 9 im Bereich des Verfahrensschrittes D.

Beim Ausblasen der Handstücke 4 von innen, wird das Wasser im Spülbehälter 2 durch die eingeblasene Druckluft stark bewegt, wodurch die Reinigungswirkung forciert wird.

Nach der Nachreinigung wird das Wasser mittels der Pumpe 29 in den Abfluß 13 abgepumpt.

Die noch vorhandene relativ hohe Temperatur der Handstücke 4 bewirkt beim Behandlungsschritt E eine Trocknung (Verdunstung) des nach dem Ablassen der Flotte an den Handstücken 4 anhaftenden Wassers. Durch Einblasen von Kaltluft nach Öffnung des Elektromagnetventils 45c in den Spülbehälter 2 werden die Handstücke 4 abgekühlt und außerdem wird die Außentrocknung forciert.

Danach können die Handstücke 4 entnommen und weitere Handstücke eingesetzt werden, und es kann ein neuer Behandlungszyklus beginnen.

Die Elektromagnetventile 66a-66f gewährleisten eine ziemlich genaue Dosierung des Pflegemittels und zwar auch in dem Fall, daß nicht alle Halterungen 3 mit Handstücken 4 besetzt werden. Durch Geschlossenhalten der Elektromagnetventile 66, die nicht besetzten Halterungen 3 zugeordnet sind, wird verhindert, daß Pflegemittel in die Zuführungsleitungen 57a-57f gelangt und bei der nächsten Besetzung der zugehörigen Halterung 3 dann mit zu großem Volumen in das zugeordnete Handstück eingeblasen wird. Hierdurch wäre nicht nur ein vermeidbarer Pflegemittelverlust vorgegeben, sondern es würde auch die Flotte vermehrt mit Pflegemittel belastet werden.

Die Steuerung der Elektromagnetventile 66 kann manuell oder auch automatisch erfolgen. Im letzteren Fall kann dem Spülbehälter 2 vorzugsweise an der Außenseite der Randwand d2 ein Sensor 101, z.B. ein Magnetschalter, zugeordnet sein, der das Vorhandensein oder Nichtvorhandensein der Trägereinheit 75 feststellt und ein Signal abgibt, das nur beim Vorhandensein der Trägereinheit 75 ein Öffnen des oder der zugehörigen Ventile 66 bewirkt. Bei der vorliegenden Ausgestaltung ist an der Außenseite des Steckverbindungsteils 76b ein Schaltmagnet 102 zur Betätigung des Magnetschalters vorgesehen.

## Patentansprüche

1. Vorrichtung (1) zum Reinigen und/oder Desinfizieren und Pflegen von ärztlichen oder zahnärztlichen Handstücken, mit wenigstens einem Träger (75), der ein Steck-Kupplungsteil (85) aufweist, mit dem das Handstück (4) mit einem korrespondierenden Steck-Kupplungsteil lösbar zusammensteckbar ist, wobei der Vorrichtung (1) eine Versorgungseinrichtung (8) mit einem Vorratsbehälter für ein Reinigungsmittel und/oder ein Desinfektionsmittel und ein Pflegemittel zugeordnet ist, von dem sich eine erste Zuführungsleitung (61, 57a-57f) zum Träger (75) erstreckt, das Steck-Kupplungsteil (85) durchsetzt und in die die mechanischen Antriebselemente aufnehmenden Hohlräume des Handstücks mündet, wobei die Versorgungseinrichtung (8) einen Druckluftleitungszweig (41a1) aufweist, der mit einer Druckluftquelle (42) verbindbar ist und mit seinem stromabwärtigen Ende stromab vom Vorratsbehälter mit der ersten Zuführungsleitung (61, 57a-57f) verbunden ist, wobei eine Steuereinrichtung vorgesehen ist, mittels der zunächst die Druckluft-Zuführung und dann die Pflegemittel-Zuführung ein- und ausschaltbar ist,
**dadurch gekennzeichnet,**
daß im Druckluftleitungszweig (41a1) stromauf seiner Verbindungsstelle mit der ersten Zuführungsleitung (61, 57a-57f) ein Rückschlagventil angeordnet ist, das in der Strömungsrichtung der Druckluft öffnet, und dem Träger (75, 75a, 75b, 75c) eine zweite Zuführungsleitung (41a2, 58a - 58 f) zugeordnet ist, die an die Druckluftquelle (42) anschließbar ist, das Steck-Kupplungsteil (85) ebenfalls durchsetzt und in der zusammengesteckten Position des Instruments (4) mit wenigstens einem Fluidkanal des Instruments (4) in Verbindung steht.

2. Vorrichtung nach Anspruch 1, dadurch gekennzeichnet, daß der Pflegemittel-Versorgungseinrichtung (8) eine eigene Druckquelle, insbesondere ein unter Druck stehender Behälter, vorzugsweise eine unter Druck stehende Sprayflasche (62), zugeordnet ist.

3. Vorrichtung nach Anspruch 1 oder 2, dadurch gekennzeichnet, daß der ersten und/oder der zweiten Zuführungsleitung (57a-57f, 58a-58f) ein insbesondere elektrischer Lufterhitzer (46) zugeordnet ist.

4. Vorrichtung nach Anspruch 3, dadurch gekennzeichnet, daß der Lufterhitzer (46) in einer mit der Druckluftquelle verbindbaren Druckluftleitung (41) angeordnet ist und die erste und die zweite Zuführungsleitung (57a-57f, 58a-58f) jeweils durch einen Druckluftleitungszweig (41a1, 41a2) miteinander verbunden sind oder mit dem Lufterhitzer (46) verbunden ist.

5. Vorrichtung nach einem der vorherigen Ansprüche, dadurch gekennzeichnet, daß eine weitere Druckluftleitung (41b) vorgesehen ist, die mit der ersten Zuführungsleitung (57a-57f) verbunden ist und wahlweise mit der Druckluftquelle (42) verbindbar ist.

6. Vorrichtung nach einem der Ansprüche 1 bis 5, dadurch gekennzeichnet, daß der Träger (75) mehrere Steck-Kupplungsteile (85a, 85b, 85c) aufweist und erste und zweite Zuführungsleitungen (57a-57f, 58a-58f) in entsprechender Anzahl vorhanden sind und jeweils mit einer gemeinsamen Mittel- oder Druckluftversorgungsleitung (61, 45a2) verbunden sind.

7. Vorrichtung nach einem der vorherigen Ansprüche, dadurch gekennzeichnet, daß mehrere Träger (75, 75a; 75b, 75c) vorgesehen sind.

8. Vorrichtung nach einem der vorherigen Ansprüche, dadurch gekennzeichnet, daß der oder die Träger (75, 75a; 75b, 75c) in einem Spülbehälter (2) vorzugsweise lösbar befestigbar ist bzw. sind, insbesondere im unteren Bereich des Spülbehälters (2) für eine stehende Anordnung des oder der Instrumente (4) oder im oberen Bereich des Spülbehälters (2) für eine hängende Anordnung des oder der Instrumente (4), wobei der Spülbehälter (2) wahlweise mit einer Flüssigkeit-Versorgungseinrichtung (5), insbesondere für Wasser, und mit einem Abfluß (13) verbindbar ist.

9. Vorrichtung nach Anspruch 8, dadurch gekennzeichnet, daß der Spülbehälter (2) mit einem Zwischenspeicher (37) verbindbar ist, der vorzugsweise höher gelegen ist, als der Spülbehälter (2).

10. Vorrichtung nach Anspruch 8 oder 9, dadurch gekennzeichnet, daß eine weitere Druckluftleitung (41c) vorgesehen ist, die in den Spülbehälter (2) mündet, vorzugsweise oberhalb seines maximalen Füllstandes (35), und die wahlweise mit der Druckluftquelle verbindbar ist.

11. Vorrichtung nach einem der Ansprüche 6 bis 10, dadurch gekennzeichnet, daß in jeder der ersten Zuführungsleitungen (57a bis 57f), die von einer gemeinsamen Versorgungsleitung (61) ausgehen, ein Sperrventil (66a bis 66f) angeordnet ist.

12. Vorrichtung nach einem der vorherigen Ansprüche, dadurch gekennzeichnet, daß ihr eine Ultraschallreinigungseinrichtung zugeordnet ist, die dann einschaltbar ist, wenn eine mit der ersten Zuführungsleitung (57a-57f) verbindbare Druckluftleitung (41a1 oder 41b) mit der Druckluftquelle (42) verbunden ist.

13. Vorrichtung nach einem der vorherigen Ansprüche, dadurch gekennzeichnet, daß der Träger (75, 75a; 75b; 75c) ein oder mehrere vorzugsweise drei im vorhandenen Raum dreieckförmig verteilt angeordnete Steck-Kupplungsteile (85) aufweist und diese Trägereinheit mittels einer Verbindungsvorrichtung, insbesondere mittels einer Steckverbindung (76) lösbar gehalten ist.

14. Vorrichtung nach Anspruch 13, dadurch gekennzeichnet, daß die wenigstens eine erste und/oder zweite Zuführungsleitung (57a-57f, 58a-58f) sich durch die Steckverbindung (76) erstrecken.

15. Vorrichtung nach einem der vorherigen Ansprüche, dadurch gekennzeichnet, daß wenigstens zwei unterschiedliche Steck-Kupplungsteile (85) vorgesehen sind, insbesondere wenigstens ein für ein Turbinenhandstück (4) und wenigstens ein für durch einen elektrischen Motor betreibbares Handstück (4).

16. Vorrichtung nach einem der vorherigen Ansprüche, dadurch gekennzeichnet, daß das oder die Steck-Kupplungsteile (85) lösbar mit dem Träger (86) verbunden sind.

17. Vorrichtung nach einem der vorherigen Ansprüche, dadurch gekennzeichnet, daß zwei oder mehrere unterschiedliche Steck-Kupplungsteile (85a, 85b, 85c) und/oder zwei oder mehrere Träger (75) mit unterschiedlichen Steck-Kupplungsteilen (85a, 85b, 85c) für unterschiedliche Instrumente (4) eines Herstellers oder eines oder mehrerer verschiedener Hersteller vorgesehen sind, wobei die Steck-Kupplungsteile (85a, 85b, 85c) und/oder die Träger (75) bezüglich ihrer Verbindungsteile (88, 76a) im Sinne eines Adapters passend ausgebildet sind und wahlweise austauschbar sind.

18. Vorrichtung nach einem der vorherigen Ansprüche, dadurch gekennzeichnet, daß für den Träger (75) und/oder das Steckkupplungsteil (85) eine Positioniervorrichtung (70) vorgesehen ist und die erste und/oder zweite Zuführungsleitung (57a-57f, 58a-58f) die Verbindungsvorrichtung (86, 88; 76) für den Träger (75) und/oder das Steck-Kupplungsteil (85) durchsetzt.

19. Vorrichtung nach einem der vorhergehenden Ansprüche, dadurch gekennzeichnet, daß die erste und/oder zweite Zuführungsleitung (57a-57f, 58a-58f) die Verbindungsvorrichtung (86, 88; 76) durchsetzt und dem in Zuführungsrichtung zuerst angeordneten Teil (86; 76) der Verbindungsvorrichtung ein Sperrventil (92) in der ersten und/oder zweiten Zuführungsleitung angeordnet ist, das bei Entfernung dieses Teils (86; 76) selbsttätig schließt.

## Claims

1. Device (1) for the cleaning and/or disinfection and care of medical or dental handpieces, with at least one carrier (75) which has a plug-in coupling part (85) with which the handpiece (4) - with a corresponding plug-in coupling part - can be releasably plugged together, whereby there is associated with the device (1) a supply arrangement (8) having a reservoir container for a cleaning agent and/or a disinfection agent and a care agent, from which a first supply line (61, 57a-57f) extends to the carrier (75), penetrates through the plug-in coupling part (85) and opens out in the hollow chamber of the handpiece accommodating the mechanical drive elements, whereby the supply arrangement (8) has a compressed air line branch (41a1) with which a compressed air source (42) can be connected and which is connected with the first supply line (61, 57a-57f) with its downstream end, downstream of the reservoir container, whereby a control apparatus is provided by means of which initially the compressed air supply and then the care agent supply can be switched on and off,
characterised in that,
there is arranged in the compressed air line branch (41a1), upstream of its connection point with the first supply line (61, 57a-57f), a non-return valve which opens in the flow direction of the compressed air, and there is associated with the carrier (75, 75a, 75b, 75c) a second supply line (41a2, 58a-58f) which can be connected to the compressed air source (42) which likewise penetrates the plug-in coupling part (85) and in the plugged-together disposition of the instrument (4) stands in connection with at least one fluid channel of the instrument (4).

2. Device according to claim 1,
characterised in that,
the care agent supply apparatus (8) has associated therewith its own pressure source, in particular a container standing under pressure, preferably a spray bottle (62) standing under pressure.

3. Device according to claim 1 or 2,
characterised in that,
the first and/or second supply line (57a-57f, 58a-58f) has associated therewith an in particular electric air heater.

4. Device according to claim 3,
characterised in that,
the air heater (46) is arranged in a compressed air line (41) which can be connected with the compressed air source and the first and the second supply lines (57a-57f, 58a-58f) are connected with each other by means of respective compressed air line branches (41a1, 41a2) or are connected with the air heater (46).

5. Device according to any preceding claim,
characterised in that,
a further compressed air line (41b) is provided, which is connected with the first supply line (57a-57f) and can be selectively connected with the compressed air source (42).

6. Device according to any of claims 1 to 5,
characterised in that,
the carrier (75) has a plurality of plug-in coupling parts (85a, 85b, 85c), and first and second supply lines (57a-57f, 58a-58f) are present in corresponding number and are each connected with a common agent or compressed air supply line (61, 45a2).

7. Device according to any preceding claim,
characterised in that,
a plurality of carriers (75, 75a; 75b, 75c) are provided.

8. Device according to any preceding claim,
characterised in that,
the carrier or carriers (75, 75a; 75b, 75c) is or are preferably releasably attachable in a rinsing container (2), in particular in the lower region of the rinsing container (2) for an up-standing arrangement of the instrument or instruments (4), or in the upper region of the rinsing container (2) for a hanging arrangement of the instrument or instruments (4), whereby the rinsing container (2) is selectively connectable with a fluid supply apparatus (5), in particular for water, and with a drain (13).

9. Device according to claim 8,
characterised in that,
the rinsing container (2) is connectable with an intermediate store (37) which is preferably located at a greater height than the rinsing container (2).

10. Device according to claim 8 or 9,
characterised in that,
a further compressed air line (41c) is provided which opens into the rinsing container (2), preferably above its maximum filling level (35), and which is selectively connectable with the compressed air source.

11. Device according to any of claims 6 to 10,
characterised in that,
in each of the first supply lines (57a-57f) which start from a common supply line (61), there is arranged a blocking valve (66a-66f).

12. Device according to any preceding claim,
characterised in that,
there is associated therewith an ultrasonic cleaning apparatus which can switched on when a compressed air line (41a1 or 41b) connectable with the first supply line (57a-57f) is connected with the compressed air source (42).

13. Device according to any preceding claim,
characterised in that,
the carrier (75, 75a; 75b; 75c) has one or more plug-in coupling parts (85), preferably three parts arranged distributed in the available space in a triangular shape, and this carrier unit is held releasably by means of a connecting device, in particular by means of a plug-in connection (76).

14. Device according to claim 13,
characterised in that,
the at least one first and/or second supply line (57a-57f, 58a-58f) extends through the plug-in connection (76).

15. Device according to any preceding claim,
characterised in that,
at least two different plug-in coupling parts (85) are provided, in particular at least one for a turbine handpiece (4) and at least one for a handpiece (4) drivable by means of an electric motor.

16. Device according to any preceding claim,
characterised in that,
the plug-in coupling part or parts (85) are releasably connected with the carrier (86).

17. Device according to any preceding claim,
characterised in that,
two or more different plug-in coupling parts (85a, 85b, 85c) and/or two or more carriers (75) having different plug-in coupling parts (85a, 85b, 85c) for different instruments (4) of a manufacturer or of one or more different manufacturers, are provided, whereby the plug-in coupling parts (85a, 85b, 85c) and/or the carriers (75) are, with regard to their coupling parts (88, 76a), suitably formed in the manner of an adapter and are selectively exchangeable.

18. Device according to any preceding claim,
characterised in that,
for the carrier (75) and/or the plug-in coupling part (85) there is provided a positioning device (70) and the first and/or second supply line (57a-57f, 58a-58f) penetrates the connection device (86, 88; 76) for the carrier (75) and/or the plug-in coupling part (85).

19. Device according to any preceding claim,
characterised in that,
the first and/or second supply line (57a-57f, 58a-58f) penetrates the connection device (86, 88; 76) and there is arranged for the first-arranged - in the supply direction - part (86; 76) of the connection device, in the first and/or second supply line, a blocking valve which upon removal of this part (86; 76) self-actingly closes.

## Revendications

1. Dispositif (1) pour nettoyer et/ou désinfecter et entretenir des pièces à main médicales ou dentaire, comprenant au moins un support (75) qui porte une partie de raccord enfichable (85) à laquelle la pièce à main (4) peut être liée de manière séparable par une partie de raccord enfichable conjuguée, un dispositif d'alimentation (8) avec un réservoir d'alimentation pour un produit de nettoyage et/ou un produit désinfectant et un produit d'entretien étant associé au dispositif (1), réservoir d'alimentation depuis lequel une première conduite d'amenée (61, 57a-57f) mène an support (75), traverse la partie de raccord enfichable (85) et débouche dans les chambres de la pièce à main qui reçoivent les éléments d'entraînement mécaniques, le dispositif d'alimentation (8) comportant un tronçon de conduite d'air comprimé (41a1) qui peut être connecté à une source d'air comprimé (42) et est reliée par son extrémité aval à la première conduite d'amenée (61, 57a-57f), en aval du réservoir d'alimentation, un dispositif de commande étant prévu, à l'aide duquel l'alimentation en air comprimé puis l'alimentation en produit d'entretien peuvent être mises en et hors service, caractérisé par le fait qu'un clapet anti-retour qui s'ouvre dans la direction d'écoulement de l'air comprimé est disposé dans la conduite d'air comprimé (41a1), en amont du point de raccordement de celle-ci à la première conduite d'amenée (61, 57a-57f), et qu'une deuxième conduite d'amenée (41a2, 58a-58f) est associée au support (75, 75a, 75b, 75c), laquelle conduite peut être connectée à la source d'air comprimé (42), traverse également la partie de raccord enfichable (85) et, dans la position enfichée de l'instrument (4) est en communication avec au moins un canal de fluide de l'instrument (4).

2. Dispositif selon la revendication 1, caractérisé par le fait qu'une source de pression propre, en particulier un réservoir sous pression, de préférence une bouteille aérosol sous pression, est associée au dispositif d'alimentation en produit d'entretien (8).

3. Dispositif selon la revendication 1 ou 2, caractérisé par le fait qu'un dispositif de chauffage d'air (46), notamment électrique, est associé à la première et/ou la deuxième conduite d'amenée (57a-57f, 58a-58f).

4. Dispositif selon la revendication 3, caractérisé par le fait que le dispositif de chauffage d'air (46) est disposé dans une conduite d'air comprimé (41) qui peut être connectée à la source d'air comprimé et que la première et/ou la deuxième conduite d'amenée (57a-57f, 58a-58f) sont reliées l'une à l'autre ou au dispositif de chauffage d'air (46) par un tronçon de conduite d'air comprimé (41a1, 41a2).

5. Dispositif selon une des revendications précédentes, caractérisé par le fait qu'il est prévu une conduite d'air comprimé (41b) supplémentaire, qui est connectée à la première conduite d'amenée (57a-57f) et peut être connectée de manière sélective à la source d'air comprimé (42).

6. Dispositif selon une des revendication 1 à 5, caractérisé par le fait que le support (75) comporte plusieurs parties de raccords enfichables (84a, 85b, 85c) et que des premières et deuxièmes conduites d'amenée (57a-57f, 58a-58f) sont prévues en nombre adapté et sont connectées chacune à une conduite d'alimentation commune de produit de nettoyage ou d'air comprimé (61, 45a2).

7. Dispositif selon une des revendications précédentes, caractérisé par le fait qu'il est prévu plusieurs supports (75, 75a, 75b, 75c).

8. Dispositif selon une des revendications précédentes, caractérisé par le fait que le ou les support(s) (75, 75a, 75b, 75c) peut ou peuvent être fixé(s), de préférence de manière démontable, dans un récipient de lavage (2), en particulier dans la partie inférieure du récipient de lavage (2) lorsque le ou les instrument(s) (4) est ou sont posé(s) et dans la partie supérieure dudit récipient de lavage lorsque le ou les instrument(s) (4) est ou sont suspendu(s), le récipient de lavage (2) pouvant être connecté de manière sélective à un dispositif d'alimentation en liquide (5), en particulier pour de l'eau, et à une évacuation (13).

9. Dispositif selon la revendication 8, caractérisé par le fait que le récipient de lavage (2) peut être connecté à un réservoir intermédiaire (37) qui, de préférence, est situé plus haut que le récipient de lavage (2).

10. Dispositif selon la revendication 8 ou 9, caractérisé par le fait qu'il une est prévu conduite d'air comprimé (41c) supplémentaire qui débouche dans le récipient de lavage (2), de préférence au dessus du niveau de remplissage (35) maximal de celui-ci, et qui peut être connectée de manière sélective à la source d'air comprimé.

11. Dispositif selon une des revendications 6 à 10, caractérisé par le fait qu'une vanne d'arrêt (66a-66f) est disposée dans chacune des premières conduites d'amenée (57a-57f) partant d'une conduite d'alimentation commune (61).

12. Dispositif selon une des revendications précédentes, caractérisé par le fait qu'un dispositif à ultrasons y est associé, lequel dispositif à ultrasons peut être mis en service lorsqu'une conduite d'air comprimé (41a1 ou 41b) pouvant être reliée à la première conduite d'amenée (57a-57f) est connectée à la source d'air comprimé (42).

13. Dispositif selon une des revendications précédentes, caractérisé par le fait que le support (75, 75a; 75b, 75c) comporte une ou plusieurs parties de raccords enfichables (85), de préférence trois parties de raccords enfichables disposées en triangle dans l'espace disponible, et que cet ensemble de support est fixé de manière démontable au moyen d'un dispositif de fixation, en particulier au moyen d'une liaison enfichable (76).

14. Dispositif selon la revendication 13, caractérisé par le fait qu la première et/ou la deuxième conduite d'amenée (57a-57f, 58a-58f) au nombre d'au moins une s'étend à travers la liaison enfichable (76)

15. Dispositif selon une des revendications précédentes, caractérisé par le fait qu'il est prévu au moins deux parties de raccords enfichables (85) différentes, en particulier au moins une partie de raccord enfichable pour une pièce à main à turbine (4) et au moins une partie de raccord enfichable pour une pièce à main (4) à moteur électrique.

16. Dispositif selon une des revendications précédentes, caractérisé par le fait que la ou les partie(s) de raccords enfichables (85) est ou sont fixées de manière démontable au support (86).

17. Dispositif selon une des revendications précédentes, caractérisé par le fait qu'il est prévu deux ou plusieurs parties de raccords enfichables (85a, 85b, 85c) différentes et/ou deux ou plusieurs supports (75) avec des parties de raccords enfichables (85a, 85b, 85c) différentes pour différents instruments (4) d'un fabricant ou d'un ou plusieurs fabricants différents, les parties de raccords enfichables (85a, 85b, 85c) et/ou les supports (75), sur le plan de leurs parties de liaison (88, 76a), étant agencées de manière adaptée et étant interechangeables.

18. Dispositif selon une des revendications précédentes, caractérisé par le fait qu'un dispositif de positionnement pour le support (75) et/ou la partie de raccord enfichable (85) est prévu et que la première et/ou la deuxième conduite d'amenée (57a-57f, 58a, 58f) traverse le dispositif de liaison (86, 88; 76) pour le support (75) et/ou la partie de raccord enfichable (85).

19. Dispositif selon une des revendications précédentes, caractérisé par le fait que la première et/ou la deuxième conduite d'amenée (57a-57f, 58a, 58f) traverse le dispositif de liaison (86, 88; 76) et qu'une vanne d'arrêt (92) est disposée dans la première et/ou la deuxième conduite d'amenée, dans la première partie (86, 76) du dispositif de liaison dans la direction d'amenée, laquelle vanne se ferme automatiquement lorsqu'on retire la partie (86, 76).
